Europäisches Patentamt

European Patent Office

Office européen des brevets

(11) Publication number: **0 377 119**
**A1**

## EUROPEAN PATENT APPLICATION

(21) Application number: 89122100.4

(51) Int. Cl.5: **A23L 1/237**

(22) Date of filing: **30.11.89**

| | |
|---|---|
| Claims for the following Contracting States: ES + GR. | (71) Applicant: **SELAKO OY**<br>**Siuntio kk**<br>**SF-02570 Siuntio(FI)** |
| (30) Priority: **07.12.88 FI 885662** | (72) Inventor: **Kurppa, Lasse**<br>**Siuntio kk**<br>**SF-02570 Siuntio(FI)** |
| (43) Date of publication of application:<br>**11.07.90 Bulletin 90/28** | |
| (84) Designated Contracting States:<br>**AT BE CH DE ES FR GB GR IT LI LU NL SE** | (74) Representative: **Prüfer, Lutz H., Dipl.-Phys.**<br>**Harthauser Strasse 25d**<br>**D-8000 München 90(DE)** |

(54) **Agent for reducing adverse effects of table salt.**

(57) The invention relates to a natural product containing dissociated K, Mg or H ions. The mixture can be used for maintenance of health as an agent reducing adverse effects of sodium chloride or table salt.

EP 0 377 119 A1

## Agent for reducing adverse effects of table salt

The invention relates to a natural product reducing the adverse effects of table salt in animals and particularly in man.

It is known that the use of industrially manufactured foodstuffs in particular leads to excessive intake of sodium chloride in man. Because of employment of such foodstuffs intakes of sodium, potassium, magnesium and calcium in particular are highly disproportionate to human vital functions. Daily dietary intakes of sodium and calcium often greatly exceed optimum values while the quantities and qualities of potassium and magnesium intakes are often inadequate for well-being. The imbalances relating to sodium and calcium result from excessive use of table salt and dairy products. The intake of magnesium and potassium has decreased, in some cases considerably because of new methods of cleaning, preparing and preserving corn and other foodstuffs. The long-term effects of the imbalances are associated with hypertension and degenerative diseases of the cardiovascular system. In particular the incidence and effects of hypertension have been found to be closely related to imbalances in intakes of sodium and potassium. The molar ratio of sodium to potassium in food should be 1:1. However, this ratio is not achieved in diets used in industrialized countries unless special measures are taken.

The ratio of magnesium to calcium should be 2:1 but low concentrations of magnesium in normal diets are a particular problem.

The optimum magnesium to potassium ratio is 4:1 for optimum intracellular electrolyte balance. If the total intake of magnesium is small the body cannot use its calcium. Calcium deficiency in turn causes certain heart diseases.

Diuretics used in the treatment of hypertension remove potassium and magnesium from the body so it is particularly important to employ natural supplements during conventional antihypertensive medication.

Various substitutes for salt have been used to reduce the adverse effects of sodium chloride, including those described in patent applications and patents FI 834309, FI 780627, DE 2305980, US 4068006, US 1262235 and NO 81522. However, all of the substitutes mentioned contain sodium chloride the intake of which is already excessive.

In the product relating to the invention the active agents are potassium chloride, which provides important potassium, magnesium sulphate to compensate for deficiencies in the usual diet, lysine hydrochloride releasing hydrogen ions, citric acid, glutamic acid and ascorbic acid to release hydrogen ions on one hand and, on the other, to prevent alkalosis, since potassium and magnesium salts have an alkalizing effect. Potato flour is used as a binding agent in the compressed tablet but can naturally be replaced by any other suitable agent such as magnesium stearate or milk powder.

Natural products having flushing effects on the kidneys can also be added to the compressed tablet, as the adding of such agents may well be in the same proportion as the basic product relating to the invention should be consumed. This ratio depends of course on the excess of sodium chloride in the diet.

The binding agent is not used in the powder form, in which case the proportions of the other agents change accordingly.

Compressed tablets also allow use of salts releasing water of crystallization and of citric acid which cause clumping in powdered dosage forms.

Potassium and magnesium salts can be replaced by oxides or sulphates. Their use in powdered dosage forms is essential to prevent clumping.

Example 1: Ingredients of compressed tablet

Potassium chloride 65%
Magnesium sulphate 28%
Potato flour 5.1%
Lysine hydrochloride 1.0%
Citric acid 0.5%
Glutamic acid 0.2%
Ascorbic acid 0.2%

## Claims

1. A composition for use as a natural product and foodstuff additive, containing potassium and magnesium ions as cations, characterized in that it contains about 65% per weight potassium chloride, about 28% per weight magnesium sulphate, about 5% per weight of a binding agent and at least one additive which releases hydrogen ions.

2. A composition according to claim 1, characterized in that it is in form of a compressed tablet.

3. A composition according to claim 1 or 2, characterized in that the additive which releases hydrogen ions is a mixture of 1% per weight lysine hydrochloride, 0.5% per weight citric acid, 0.2% per weight glutamic acid and 0.2% per weight asorbic acid.

4. A mixture according of any of claims 1 to 3, characterized in that it contains natural substances

3 EP 0 377 119 A1 4

which have flushing effects on the kidneys.

5. Use of a composition according to any of
Claims for the foregoing Contracting States: GR, ES

1. A process for preparing a composition for use as a natural product or foodstuff additive, characterized in that a mixture is prepared containing potassium and magnesium ions as cations, characterized in that it contains about 65% per weight potassium chloride, about 28% per weight magnesium sulphate, about 5% per weight of a binding agent and at least one additive which releases hydrogen ions.

2. A process according to claim 1, characterized in that the composition is in form of a compressed tablet.

3. A process according to claim 1 or 2, characterized in that the additive which releases hydrogen ions is a mixture of 1% per weight lysine hydrochloride, 0,5% per weight citric acid, 0.2% per weight glutamic acid and 0.2% per weight asorbic acid.

4. A process according to any of claims 1 to 3, characterized in that natural substances which have flushing effects on the kidneys are added.

5. Use of a composition prepared according to any of claims 1 to 4 as a foodstuff additive.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 068 006  (G.J. MORITZ)<br>* abstract; claims 1,8 *<br>--- | 1,3 | A 23 L    1/237 |
| A | FR-A-2 196 151  (G.S. GRIMBERG et al.)<br>* claims 1,2 *<br>--- | 1 | |
| A | US-A-2 471 144  (E.D. DAVY)<br>* column 2, examples; claims 1-3 *<br>--- | 1 | |
| A | EP-A-0 124 254  (NABISCO BRANDS INC.)<br>* abstract; claim 1 *<br>----- | 1 | |
| | | | **TECHNICAL FIELDS SEARCHED (Int. Cl.5)**<br><br>A 23 L    1/00 |

The present search report has been drawn up for all claims

| Place of search | Date of completion of the search | Examiner |
|---|---|---|
| BERLIN | 01-03-1990 | SCHULTZE D |

EPO FORM 1503 03.82 (P0401)

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document